# EUROPÄISCHE PATENTSCHRIFT

(11) **EP 2 519 172 B1**
(45) Veröffentlichungstag und Bekanntmachung des Hinweises auf die Patenterteilung: **05.04.2017**
(21) Anmeldenummer: 10805697.9
(22) Anmeldetag: 30.12.2010
(51) Int. Cl.: A61B 90/00

(54) **PUNKTIONSNADELSYSTEM**
PUNCTURE NEEDLE SYSTEM
SYSTÈME D'AIGUILLE DE PONCTION

(30) Priorität: 31.12.2009 DE 102009060921
(43) Veröffentlichungstag der Anmeldung: 07.11.2012
(73) Patentinhaber: POLYDIAGNOST ENTWICKLUNGS-, PRODUKTIONS-, VERTRIEBS-, UND SERVICE GESELLSCHAFT MBH, 85276 Pfaffenhofen (DE)
(72) Erfinder: SCHAAF, Hansgeorg, 85293 Reichertshausen (DE)
(74) Vertreter: Winter, Brandl, Fürniss, Hübner, Röss, Kaiser, Polte - Partnerschaft mbB
(86) Internationale Anmeldenummer: PCT/EP2010/007993
(87) Internationale Veröffentlichungsnummer: WO 2011/079958

(56) Entgegenhaltungen:
- WO-A1-96/01132
- WO-A1-02/083003
- WO-A1-2009/109873
- DE-U1-202007 011 781
- US-A- 4 254 762
- US-A- 4 736 733
- US-A- 5 336 176
- US-A- 5 738 628
- US-A1- 2006 241 450
- US-A1- 2006 270 988
- US-A1- 2009 264 913
- US-A1- 2009 270 819

## Beschreibung

Die Erfindung betrifft ein Punktionsnadelsystem zur Punktion eines Organs, insbesondere einer Niere.

Nach epidemiologischen Studien steigt die Zahl von Patienten mit Harnstein-/Nierensteinerkrankungen stetig an. Diese Zunahme wird mit veränderten Ernährungs- und Lebensgewohnheiten erklärt. Obwohl viele Harn-/Nierensteine den Körper auf natürliche Weise ohne Eingriff passieren können, ist in einer Vielzahl von Fällen zur Vermeidung von Komplikationen ein Eingriff zur Entfernung/Zerkleinerung der Harnsteine erforderlich.

Zur nichtinvasiven Zerkleinerung von Nierensteinen wird die extrakorporale Stoßwellen-Lithotripsie verwendet, die in den 80er Jahren entwickelt wurde. Dabei werden über einen Lithotripter ausserhalb des Körpers Druckwellen erzeugt, die in Richtung auf den Nierenstein fokussiert sind und diesen in eine Vielzahl kleinere Partikel fragmentieren. Diese werden dann auf natürliche Weise aus dem Harntrakt ausgeschieden.

Nachteil dieses Verfahrens ist, dass es beim Abgang dieser Steinfragmente zur Koliken kommen kann und dass eine vollständige Entfernung der Steinfragmente nicht gewährleistet ist.

Insbesondere in Fällen, in denen sich der Nierenstein in einer für eine extrakorporale Stoßwellen-Lithotripsie nicht zugänglichen Lage befindet oder wenn er zu groß für eine derartige Behandlung ist, findet die so genannte perkutane Nephrolithotomie (PCNL) Anwendung. Dabei wird über eine Punktionsnadel ein Tunnel direkt zur Niere ausgebildet und durch diesen Tunnel ein Nephroskop eingesetzt, so dass der Stein direkt lokalisiert und entfernt wird. Dafür können starre und flexible Endoskope eingesetzt werden. Als besonders geeignet hat sich das von der Anmelderin unter der Marke "Polyscope^{®}" vertriebene flexible Endoskop erwiesen.

In dem Fall, in dem der Nierenstein zu groß für eine direkte Entnahme mittels eines Zängchens oder Körbchens ist, muss dieser zunächst fragmentiert werden. Dies kann durch Laser-, Ultraschall-, pneumatische oder elektrohydraulische Lithotripsie erfolgen. Einzelheiten zu diesen Verfahren sind dem Aufsatz "Epidemiologische, instrumentelle Therapie und Metaphylaxe des Harnsteinleidens"; Stephan C. Müller; Deutsches Ärzteblatt, Jg. 101, Heft 19, 7. Mai 2004 entnehmbar.

Unter dem Produktnamen "Lithoclast^{®}" wird ein pneumatischer Lithotripter angeboten, der bei sehr geringem Außendurchmesser (3 Fr) eine effektive Zerkleinerung ermöglicht. Bei der Weiterentwicklung LITHOCLAST®MASTER werden drei elementare Lithotripsiefunktionen, d.h. ein pneumatischer Lithotripter der vorbeschriebenen Bauweise, ein Ultraschall-Lithotripter und ein Absaugsystem kombiniert, um die Nierensteine bzw. Nierensteinfragmente entfernen zu können.

In dem Fall, in dem die Steine im mittleren und unteren Ureter angeordnet sind, ist unter Umständen keine Punktion erforderlich, da dann ein Ureteroskop durch die Harnröhre hindurch in den Ureter eingeführt werden kann. Diese Ureteroskopie hat jedoch den Nachteil, dass nur vergleichsweise kleine Steine über Körbchen oder Zängchen direkt entnommen werden können und bei größeren Steinen wiederum eine Lithotripsie erforderlich ist, wobei die Steinfragmente dann ausgespült werden müssen und im Ureter hängen bleiben können - bei größeren Steinen und/oder ungünstigen Lagen der Steine im Harntrakt wird daher weiterhin die minimalinvasive PCNL bevorzugt.

Der wichtigste Schritt zur Vermeidung von Komplikationen bei der PCNL ist dabei die anatomisch korrekte Ausbildung des Kanals zu dem Nierenstein über die Punktionsnadel. Bei der herkömmlichen PCNL wird die korrekte Position der Punktionsnadel mit Bezug dem zu entfernenden Nierenstein über Ortungshilfsmittel, wie beispielsweise Röntgenkontrolle, Fluoroskopie (Kontrastmittel) oder Ultraschall kontrolliert - die Qualität des Zugangs kann jedoch erst dann überprüft werden, wenn der mit den herkömmlichen Techniken erstellte Kanal bougiert ist und das Nephroskop eingesetzt ist. Nach der Ausbildung dieses bougierten Zugangs ist allerdings eine nachträgliche Korrektur nicht oder nur mit erheblichem Aufwand möglich.

US 4 254 762 A offenbart ein Punktionsnadelsystem zur Punktion eines Organs oder eines Gefäßes, mit einer Punktionskanüle, die von einer Optik durchsetzt ist, wobei die Punktionskanüle einen Außendurchmesser zwischen 1.7 und 10 mm hat. Dem gegenüber liegt der Erfindung die Aufgabe zugrunde, ein Punktionsnadelsystem zu schaffen, mit dem ein Zugang mit hoher Präzision herstellbar ist.
Diese Aufgabe wird durch ein Punktionsnadelsystem mit den Merkmalen des Patentanspruches 1 gelöst.

Erfindungsgemäß ist das Punktionsnadelsystem als Baugruppe mit einem Arbeitsschaft ausgeführt, in den eine Punktionskanüle eingesetzt ist, deren Spitze aus dem Arbeitsschaft auskragt und die von einer Optik durchsetzt ist. Das Punktionsnadelsystem ist somit als "sehende Punktionsnadel" ausgeführt, wobei über die Optik auf einfache Weise kontrolliert werden kann, ob die Punktionskanüle richtig positioniert ist.

Auf diese Weise ist es möglich, die Qualität des Zugangs zum punktierten Gefäß bzw. zum punktierten Organ, beispielsweise einem Harntrakt bereits während der Punktion ohne die eingangs genannten Ortungshilfsmittel zu überprüfen. Darüber hinaus ist es bereits während der Punktion möglich, den eventuell im vorgesehen Punktionskanal liegenden Gefäßen oder dem Darm auszuweichen, so dass deren Perforation verhindert wird.

Die Verwendung des Arbeitsschaftes ermöglicht es, ohne weiteres Aufbougieren nach dem Entnehmen der Punktionsnadel zur Zerkleinerung einen Lithotripter oder dergleichen anzusetzen. Prinzipiell ist es auch möglich, in oder an diesen Arbeitsschaft ein herkömmliches Endoskop zur Entfernung des Nierensteins anzusetzen. Alternativ kann auch die so genannte "Rendezvoustechnik" eingesetzt werden, bei der zum Zertrümmern eines Nierensteins zunächst von außen durch die Bauchdecke hindurch punktiert und anschließend mittels eines Lithotripters der Nierenstein fragmentiert wird. Parallel kann ein flexibles Ureteroskop über den Harnleiter eingeführt werden, um die Steinfragmente oder den Nieren-/Harnleiterstein schonend abzusaugen oder über ein Körbchen, Zängchen zu entnehmen.

Auf diese Weise kann eine minimalinvasive endoskopische Operation mit geringsten Beeinträchtigungen für den Patienten durchgeführt werden.

Die "sehende Punktionsnadel" ist im Stand der Technik ohne jegliches Vorbild, da dort allenfalls die Nephroskope oder die Ureteroskope mit einer Optik versehen werden, die Punktion jedoch stets blind unter Hinzunahme von Ortungshilfsmitteln durchgeführt wird. Diese ermöglichen lediglich eine 2D-Bildauswertung. Prinzipiell kann bei dem erfindungsgemäßen Punktionsnadelsystem auch eine 3D-Optik mit wesentlich exakteren Auswertemöglichkeiten verwendet werden.

Gemäß der Erfindung wird ein Arbeitsschaft mit einem sehr geringen Außendurchmesser von weniger als 5 Fr verwendet.

Die mechanische Verbindung zwischen dem Arbeitsschaft und der Punktionskanüle erfolgt über eine lösbare Kupplung, vorzugsweise über ein Luerlock-System.

Bei einem Ausführungsbeispiel der Erfindung sind die Punktionskanüle und der Arbeitsschaft an einen Handgriff angesetzt, der mit einem Y-Stück zum Einsetzen der Optik ausgeführt ist.

Dieser Handgriff kann mit einem T-Adapter zum axialen Ansetzen einer Einrichtung zum Desintegrieren eines Steins durch Ultraschall, Laserenergie oder durch mechanische Einwirkung ausgeführt sein. Ein radialer Anschluss dieses T-Adapters kann als Spülanschluss ausgeführt werden.

Erfindungsgemäß wird es bevorzugt, dass die Optik mit einem Längenausgleich (Shifter) ausgeführt ist, über den die Optik mit Bezug zur Spitze der Punktionsnadel ausgerichtet werden kann, so dass eine Adaption an unterschiedliche Schaftlängen möglich ist.

Erfindungsgemäß wird es bevorzugt, wenn die Optik mit einem Öffnungswinkel von >70°, vorzugsweise mit einem Öffnungswinkel von 120° ausgeführt ist.

Die Auflösung sollte bei mehr als 6000 Pixel, vorzugsweise bei etwa 10000 Pixel liegen.

Die Handhabung des Punktionsnadelsystems ist besonders einfach, wenn die Punktionskanüle, der Arbeitsschaft, der Handgriff und/oder auch der Optik-Shifter als Einwegbauteile (disposable) ausgeführt sind. Alternativ kann es vorgesehen werden, zumindest den Handgriff autoklavierbar auszuführen.

In dem Fall, in dem der Durchmesser des Arbeitsschaftes nicht ausreicht, um die vorgesehene Einrichtung zur Desintegration des Steines aufzunehmen, kann in den mittels der vorbeschriebenen Punktionskanüle ausgebildeten Kanal ein größerer Arbeitsschaft mit einem Obturator zum Bougieren dieses Zugangs eingeführt werden. Dieser größere Arbeitsschaft mit Obturator kann über einen Führungsdraht in den Zugang eingesetzt werden. Der Führungsdraht kann entweder nach dem Herausziehen der Punktionskanüle und des kleineren Arbeitsschaftes oder bereits mit der Punktionskanüle eingeführt werden.

An diesen größeren Arbeitsschaft kann dann ein Lithotripter der eingangs beschriebenen Art mit Ultraschalleinheit/mechanischer Einheit und Absaugsystem (LiTHOCLAST^{®}MASTER) angesetzt werden, so dass auch große Nierensteine schonend desintegriert und abgesaugt werden können.

Bevorzugte Ausführungsbeispiele der Erfindung werden im Folgenden anhand schematischer Zeichnungen näher erläutert. Es zeigen:
Figur 1 eine schematische Darstellung eines erfindungsgemäßen Punktionsnadelsystems;
Figur 2 eine Darstellung eines Handgriffs des Punktionsnadelsystems aus Figur 1;
Figur 3 Einzelteile des Handgriffs aus Figur 2;
Figur 4 einen alternativen autoklavierbaren Handgriff;
Figur 5 eine Einzeldarstellung eines Arbeitsschaftes des Punktionsnadelsystems aus Figur 1;
Figur 6 eine Punktionskanüle des Punktionsnadelsystems aus Figur 1;
Figur 7 eine Optik des Punktionsnadelsystems und
Figur 8 einen Obturator für den Arbeitsschaft gemäß Figur 8.

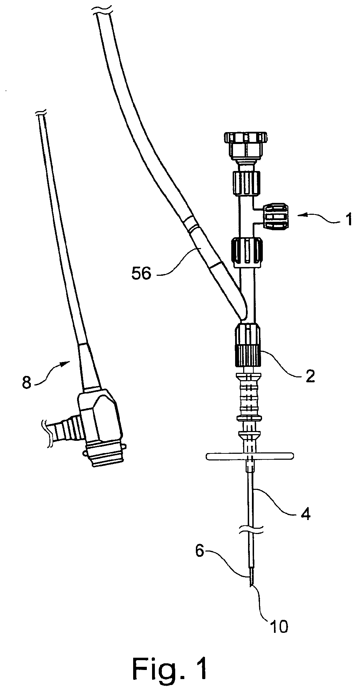

Figur 1 zeigt eine Teilansicht eines erfindungsgemäßen modularen Punktionsnadelsystems 1. Dieses besteht im Prinzip aus einem Handgriff 2, an den ein Arbeitsschaft 4 und eine letzteren durchsetzende Punktionskanüle 6 angesetzt sind, deren distales Ende aus der Mündung des Arbeitsschaftes 4 hervorsteht und dort mit einer scharfen Spitze 10 ausgeführt ist, um einen Zugang zu einem Organ, beispielsweise einem Nierenkelch oder einem Harnleiter eines Harntrakts herzustellen. Eine Besonderheit des dargestellten Punktionsnadelsystems 1 besteht darin, dass eine Optik 8 vorgesehen ist, deren Optikkopf im Bereich der Spitze 10 der hohlen Punktionsnadel 6 mündet und über die derjenige Bereich optisch erfassbar ist, in dem sich diese Spitze 10 des Punktionsnadelsystems befindet. Auf diese Weise ist eine Online-Kontrolle der Punktionsnadelposition möglich, so dass die korrekte Lage des hergestellten Zugangs überprüft werden kann und eine versehentliche Verletzung von Gefäßen, anderer Organe oder des Darms ausgeschlossen ist. Das dargestellte Punktionsnadelsystem wird vorzugsweise bei der minimalinvasiven perkutanen Nephrolitholapaxie (PCNL) verwendet, um beispielsweise von der Bauchdecke her einen Zugang zum Nierenhohlsystem herzustellen, um Nierensteine oder Harnleitersteine zu lokalisieren und zu desintegrieren und anschließend über ein in den punktierten Kanal eingesetztes Nephroskop bzw. ein über den Harnleiter eingeführtes Ureteroskop aktiv zu entfernen.

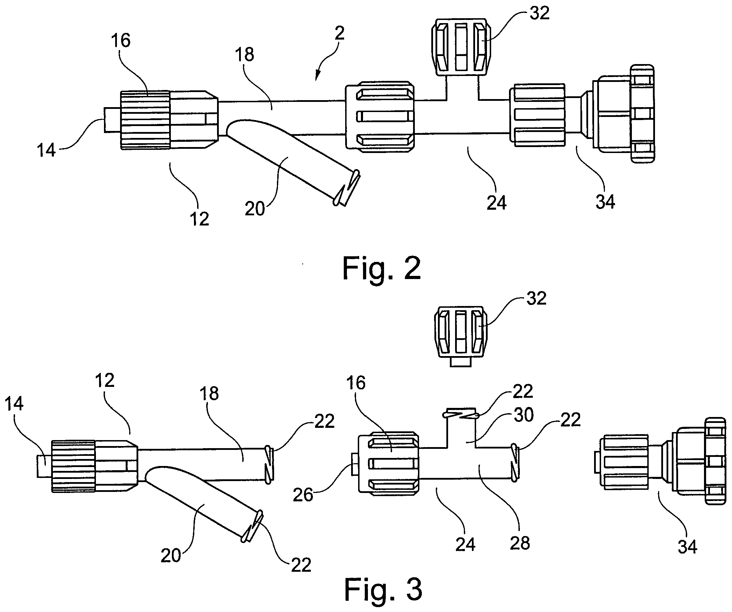

Die einzelnen Bauelemente des Punktionsnadelsystems 1 gemäß Figur 1 werden anhand der Figuren 2 bis 8 näher erläutert.

Figur 2 zeigt den Handgriff 2 und Figur 3 dessen Einzelteile. Demgemäß hat der Handgriff 2 einen Y-Adapter 12, an dessen kanülenseitigen Endabschnitt 14 ein Außenteil 16 einer Luer-Kupplung ausgebildet ist, während am distalen Axialanschluss 18 und am Y-Anschluss 20 jeweils ein Innenteil 22 einer entsprechenden Luer-Kupplung ausgebildet ist. Gemäß der Darstellung in Figur 2 ist an den Y-Adapter ein T-Adapter 24 angesetzt, wobei dessen zum Y-Adapter 12 weisender Anschluss 26 mit einem Außenteil 16 einer Luer-Kupplung und der andere axiale Anschluss 28 sowie ein Radialanschluss 30 in entsprechender Weise jeweils mit einem Luer-Innenteil 22 ausgeführt sind.

Auf dem Radialanschluss 30 ist eine Luer-Kappe 32 aufgesetzt. Falls es eine Indikation erfordert, kann an diesen Radialanschluss 30 beispielsweise eine nicht dargestellte Spülleitung angeschlossen werden. An den distalen axialen Anschluss 28 ist ein Adapter, beispielsweise ein Touhy-Borst-Adapter 34 angesetzt, über den beispielsweise eine Einrichtung zum Desintegrieren eines Nierensteins angesetzt werden kann. Diese Einrichtung kann beispielsweise eine Laserfiber sein, die über den Adapter 34 angesetzt und bis zur Spitze 10 vorgeschoben wird, so dass der Nierenstein mit Laserenergie beaufschlagt werden kann - dieser Vorgang kann dabei über die Optik 8 überwacht werden. Deren Material ist so ausgelegt, dass es der bei Laserbeaufschlagung frei werdenden Wärmeenergie Stand hält. Als besonders gut geeignet haben sich dabei mit Nitinol beschichtete Optiken gezeigt.

Anstelle einer Laserfiber kann selbstverständlich auch einer der eingangs beschriebenen Lithotripter, beispielsweise das mit sehr geringem Außendurchmesser ausgeführte System LITHOCLAST^{®} der Firma EMS (www. ems-Company.com) eingesetzt werden.

Der vorbeschriebene Handgriff 2 hat einen sehr einfachen Aufbau, wobei die Adapter 12, 24, 34 vorzugsweise aus Kunststoff, beispielsweise im Spritzgießverfahren als disposable Bauelemente hergestellt werden.

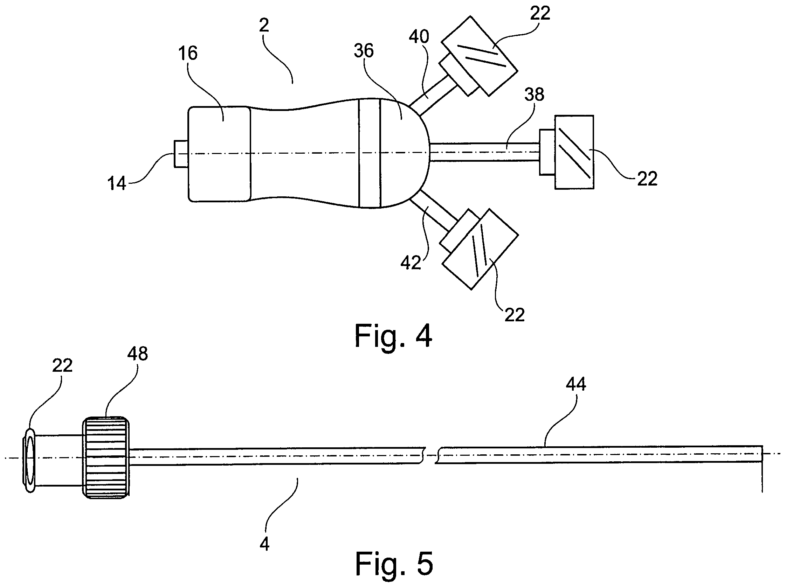

Für den Fall, dass ein wieder verwendbarer Handgriff verwendet werden soll, kann dieser gemäß Figur 4 aus einem autoklavierbaren Material, beispielsweise aus Edelstahl hergestellt werden. Dieser Handgriff 2 hat an seinem mit Bezug zur Punktionskanüle proximalen Anschluss 14 ein Luer-Außenteil 16, am distalen Endabschnitt 36 des Handgriffs 2 sind ein axialer Anschluss 38 und zwei Y-Anschlüsse 40, 42 ausgebildet, die jeweils mit einem Luer-Innenteil 22 ausgeführt sind, so dass in entsprechender Weise zur Variante gemäß Figur 2 an den axialen Anschluss 38 eine Einrichtung zum Desintegrieren des Nierensteins, an einen der Y-Anschlüsse 42 die Optik 8 und an den anderen Y-Anschluss 40 eine Spülleitung oder eine Verschlusskappe 32 angesetzt werden kann. Dieser Handgriff 2 hat eine wesentliche kürzere Axiallänge als der in Figur 2 dargestellte Handgriff 2. Prinzipiell ist es auch möglich, diesen kurzen Handgriff 2 im Spritzgießverfahren als Disposable herzustellen.

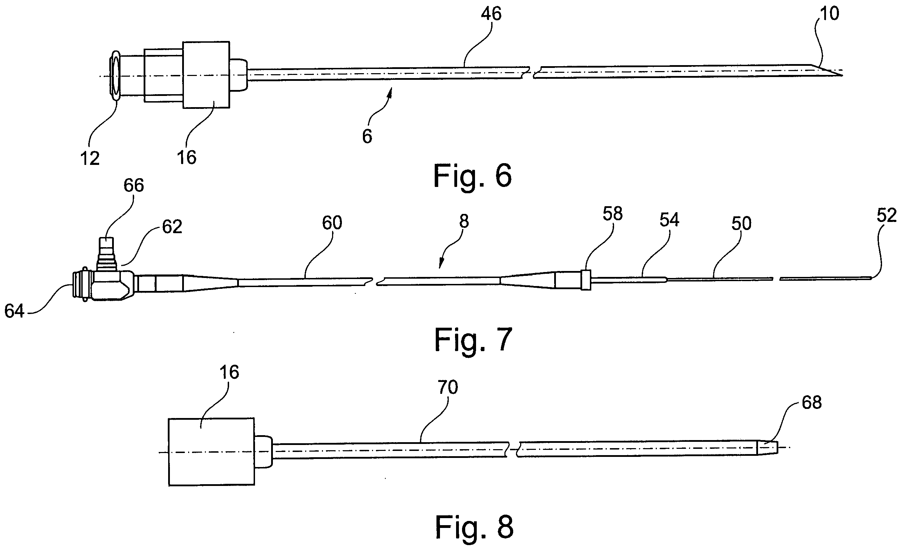

Die Figuren 5 und 6 zeigen Einzelzeichnungen der an den proximalen Anschluss 14 angesetzten Punktionskanüle 6 (Figur 6) und des Arbeitsschaftes 4 (Figur 5). Beim dargestellten Ausführungsbeispiel hat der Arbeitsschaft 4 einen Schaft 44 (Figur 5), an dem ein Innenteil 22 und im Abstand dazu ein Rändelgriff 48 einer Luer-Kupplung ausgebildet sind. Beim dargestellten System beträgt der Außendurchmesser des Schaftes etwa 4,8 Fr (1,6 mm), der Innendurchmesser beträgt in etwa 4,2 Fr (1,4 mm).

Die in Figur 6 dargestellte Punktionskanüle 6 hat eine Kanüle 46, an deren in Figur 6 rechts liegenden Endabschnitt die durch eine Anschrägung ausgebildete Spitze 10 vorgesehen ist. Der Außendurchmesser der Kanüle 46 beträgt beim dargestellten Ausführungsbeispiel in etwa 3,9 Fr (1,3 mm), so dass die Kanüle 46 in den Schaft 44 eingesetzt werden kann. Der Innendurchmesser der Kanüle 46 ist beim dargestellten Ausführungsbeispiel etwa 3,3 Fr (1,1 mm), so dass genügend Platz zum Einsetzen der Optik 8 verbleibt.

An dem in Figur 6 linken Endabschnitt der Punktionskanüle 6 sind wiederum ein Außenteil 16 und ein Innenteil 22 einer Luer-Kupplung ausgebildet. Gemäß Figur 1 wird die Kanüle 46 in der Darstellung gemäß Figur 5 von links her durch die Luer-Lock-Elemente 22, 16 hindurch in den Schaft 44 des Arbeitsschaftes 4 eingeschoben und dabei das Luer-Außenteil 16 der Punktionskanüle 6 mit dem Luer-Innenteil 22 des Arbeitsschaftes 4 gekuppelt, wobei das Eindrehen mit Hilfe des am Arbeitsschaft 4 ausgebildeten Rändelgriffs 48 erfolgt.

In diesem Zustand steht dann das Luer-Innenteil 22 der Punktionsnadel 6 axial über den Arbeitsschaft 44 hinaus und kann dann entsprechend mit dem Luer-Außenteil 16 des Y-Adapters 12 gekuppelt werden, so dass die Punktionskanüle 6 und der Arbeitsschaft 4 fluiddicht und mechanisch stabil mit dem Handgriff 2 verbunden sind.

Figur 7 zeigt den Aufbau der Optik 8 des Punktionsnadelsystems 1. Der Grundaufbau dieser Optik 8 ist aus dem Polyscope^{®}-System der Anmelderin bekannt, so dass hier nur die zum Verständnis der Erfindung wesentlichen Bauelemente erläutert werden. Hinsichtlich weiterer Details der Optik sei beispielhaft auf die DE 20 2007 011 781 U1 der Anmelderin verwiesen. Eine derartige Optik 8 hat ein hochflexibles Bündel geordneter Bild- und Lichtfasern, über die der zu untersuchende Bereich beleuchtet und Bildinformationen einem Okular- oder einem angeschlossenen Bildaufzeichnungsgerät zugeleitet werden. Beim dargestellten Ausführungsbeispiel ist die Optik 8 so ausgelegt, dass sich eine Auflösung von etwa 6000 Pixel ergibt. Die in der Figur 7 nicht dargestellten Bild- und Lichtfasern sind bei dem dargestellten Ausführungsbeispiel in einem Kanülenrohr 50 angeordnet, wobei der Mündungsbereich 52 der Optik so ausgelegt ist, dass sich ein Öffnungswinkel von etwa 70° ergibt. Bei einer derartigen Optik 8 hat das Kanülenrohr 50 einen Außendurchmesser von etwa 0,55 mm, so dass dieses Kanülenrohr 50 gemeinsam mit der beispielhaft genannten Laserfiber in die Kanüle 46 eingesetzt werden kann. Das Kanülenrohr 50 geht in ein Führungsrohr 54 über, das mit einem Optik-Shifter 56 (siehe Figur 1) zusammen wirkt. Dieser ist bei der dargestellten Variante auf den Y-Anschluss 20 des Handgriffs 2 aufgesetzt und mit diesem über eine Luer-Lock-Kupplung verbunden. Die Optik 8 wird mit dem Kanülenrohr 50 und dem Führungsrohr 54 in den Shifter 56 und über den Y-Anschluss 20 in die Kanüle 46 eingeführt und dabei solange eingeschoben bis die Mündung 52 im Bereich der Spitze 10 der Kanüle 46 angeordnet ist. Diese Position kann beispielsweise über die Bildauswertung überwacht werden. Sobald diese Sollposition erreicht wird, wird eine Klemmschraube des Optik-Shifters 56 mit dem Führungsrohr 54 verspannt, so dass die Optik lagepositioniert ist. An das Führungsrohr 54 schließt sich eine Knickschutzhülse 58 an, die als Endstück eines Optikschutzschlauches 60 ausgeführt ist. An dessen distalen Endabschnitt ist ein Optikgrundkörper 62 mit einem Beleuchtungsanschluss 64 und einem Okularanschluss 66 angeordnet. An diesen kann ein Okular, beispielsweise ein Zoomokular angesetzt werden. Über den Beleuchtungsanschluss 64 werden die Lichtleiter der Optik 8 an eine Lichtquelle angeschlossen. Das Zoomokular ermöglicht es, trotz des geringen Außendurchmessers von etwa 0,55 mm des Kanülenrohrs 50 das Bild mit einem hinreichenden Durchmesser von mehr als 11 cm abzubilden.

Hinsichtlich weiterer Einzelheiten der Optik 8 und des Optik-Shifters wird auf die genannte DE 20 2007 011 781 U1 und die DE 10 2005 08 825 B3 verwiesen.

Wie bereits erwähnt, kann mit der beschriebenen Optik eine Abbildung mit einer Auflösung von 6000 Pixel bei einem Öffnungswinkel der Optik von 70° erfasst werden - falls höhere Anforderungen an die Bildqualität bestehen, kann eine Optik mit einer höheren Auflösung von beispielsweise 10.000 Pixel und einem Öffnungswinkel von 120° verwendet werden. Eine derartige Optik hat einen Kanülenrohrdurchmesser von etwa 0,93 mm, so dass sie nicht oder nur schwierig gemeinsam mit einer Laserfiber in die Kanüle 46 eingeschoben werden kann. In diesem Fall muss ein Arbeitsschaft 4 mit einem größeren Durchmesser verwendet werden, es kann jedoch auch vorteilhaft sein, wenn ein besonderer Handgriff vorgesehen wird, der das Einsetzen von schwergängigen Komponenten in die vergleichsweise enge Kanüle 46 ermöglicht.

In dem Fall, in dem mechanisch lithotripsiert und die Steinfragmente abgesaugt werden sollen, ist in der Regel eine Sonde mit einem Außendurchmesser erforderlich, der größer ist als der Innendurchmesser der dargestellten Kanüle 46.

Dazu wird das vorbeschriebene Punktionsnadelsystem 1 aus dem Kanal heraus gezogen und ein Führungsdraht eingesetzt und entlang dieses Führungsdrahtes ein Arbeitsschaft mit größerem, für den Lithotripter, beispielsweise LITHOCLAST^{®}MASTER, geeigneten Innendurchmesser in den Kanal eingeschoben. Ein derartiger Arbeitsschaft 4 hat im Prinzip die in Figur 5 dargestellte Geometrie, wobei der Außendurchmesser des Schaftes etwa 8,1 Fr (2,7 mm) oder 10,5 Fr (3,5 mm) beträgt, der Innendurchmesser beträgt dann entsprechend 6,9 Fr bzw. 9 Fr. Dieser Innendurchmesser reicht aus, um einen für die Desintegration und die Absaugung geeigneten Lithotripter anzusetzen. In diesem Arbeitsschaft 4 mit vergleichsweise großem Durchmesser wird ein Obturator 70 eingesetzt, der an seinem in Figur 8 linken Endabschnitt ein Luer-Außenteil 16 hat, das in Eingriff mit dem Innenteil 22 an den Arbeitsschaft 4 gebracht werden kann. In diesem gekoppelten Zustand steht ein kegelig verjüngtes Distalende 68 des Obturators 70 aus dem Schaft 44 des Arbeitsschaftes 4 heraus, so dass der zuvor ausgebildete Kanal bougiert wird und der Arbeitsschaft 44 entlang des Führungsdrahtes zum Nierenstein geschoben werden kann. Dieser Einschubvorgang kann wiederum über die Optik 8 überwacht werden, die in den Arbeitsschaft 44 eingesetzt werden kann.

Nach dem Erreichen der Sollposition wird der Obturator 70 aus dem Arbeitsschaft 44 heraus gezogen und der Lithotripter angesetzt, wobei die Optik 8 in ihrer Position verbleiben kann. Dabei ist die Optik 8 so ausgelegt, dass die in direkten Kontakt mit dem Lithotripter stehenden Bereiche der Optik 8 durch die Vibrationen des Lithotripters nicht beschädigt werden können.

Nach der Punktion werden der Handgriff 2 gemäß Figur 2, der Optik-Shifter 56, die Punktionskanüle 6 und ggf. auch der Arbeitsschaft 4 als Disposable entsorgt, während die sonstigen Bauelemente nach einer Dekontamination wieder verwendbar sind.

D.h. bei einem derartigen System ist der Optik-Shifter 56 praktisch in den Y-Adapter 18 integriert und wird gemeinsam mit diesem entsorgt.

Wie bereits erwähnt, kann auch eine dekontaminierbare Lösung aus autoklavierbarem Material verwendet werden.

Offenbart ist ein Punktionsnadelsystem mit einer Optik.

### Bezugszeichenliste:

- 1: Punktionsnadelsystem
- 2: Handgriff
- 4: Arbeitsschaft
- 6: Punktionskanüle
- 8: Optik
- 10: Spitze
- 12: Y-Adapter
- 14: proximaler Endabschnitt
- 16: Außenteil (Luer-Kupplung)
- 18: axialer Anschluss
- 20: Y-Anschluss
- 22: Innenteil (Luer-Kupplung)
- 24: T-Adapter
- 26: axialer Anschluss
- 28: distaler Axialanschluss
- 30: Radialanschluss
- 32: Luer-Kappe
- 34: Touhy-Borst-Adapter
- 36: distaler Endabschnitt
- 38: axialer Anschluss
- 40: Y-Anschluss
- 42: Y-Anschluss
- 44: Schaft
- 46: Kanüle
- 48: Rändelgriff
- 50: Kanülenrohr
- 52: Mündung
- 54: Führungsrohr
- 56: Optik-Shifter
- 58: Knickschutzhülse
- 60: Optikschutzschlauch
- 62: Optikgrundkörper
- 64: Beleuchtungsanschluss
- 66: Okularanschluss
- 68: Distalende
- 70: Obturator

## Patentansprüche

1. Punktionsnadelsystem zur Punktion eines Organs oder eines Gefäßes, insbesondere einer Niere, mit einer Punktionskanüle (6), die in einen Arbeitsschaft (4) eingesetzt und von einer Optik (8) durchsetzt ist, wobei der Arbeitsschaft (4) einen Außendurchmesser von weniger als 5 Fr hat.

2. Punktionsnadelsystem nach Patentanspruch 1, wobei der Arbeitsschaft (4) und die Kanüle (6) über eine Kupplung, vorzugsweise eine Luer-Kupplung (16, 22) verbunden sind.

3. Punktionsnadelsystem nach einem der vorhergehenden Patentansprüche, mit einem Handgriff (2), der an die Kanüle (6) und/oder den Arbeitsschaft (4) angesetzt ist und einen Y-Adapter (12) zum Ansetzen der Optik (8) hat.

4. Punktionsnadelsystem nach Patentanspruch 3, mit einem T-Adapter (24) zum axialen Ansetzen an das Y-Stück (12), mit einem Radialanschluss (30) als Spülanschluss.

5. Punktionsnadelsystem nach Patentanspruch 4, mit einem Axialanschluss (28) für eine Laserfiber oder dergleichen.

6. Punktionsnadelsystem nach einem der vorhergehenden Patentansprüche, wobei der Optik (8) ein Optik-Shifter (56) zugeordnet ist.

7. Punktionsnadelsystem nach einem der vorhergehenden Patentansprüche, wobei die Optik (8) mit einem Öffnungswinkel von mehr als 70° ausgeführt ist.

8. Punktionsnadelsystem nach einem der vorhergehenden Patentansprüche, wobei die Optik (8) mit einer Auflösung von mehr als 6000 Pixeln ausgeführt ist.

9. Punktionsnadelsystem nach einem der vorhergehenden Patentansprüche, wobei der Arbeitsschaft (4), die Punktionskanüle (6) und der Handgriff (2) sowie der Optik-Shifter (56) disposable ausgeführt sind.

10. Punktionsnadelsystem nach einem der Patentansprüche 1 bis 9, wobei der Handgriff (2) autoklavierbar ausgeführt ist.

11. Punktionsnadelsystem nach einem der vorhergehenden Patentansprüche, mit einem weiteren Arbeitsschaft (4) mit größerem Durchmesser und einem Obturator (70) zum Bougieren des mit der Punktionskanüle (6) hergestellten Zugangs und mit einem Führungsdraht zum Führen des Arbeitsschaftes (4).

12. Punktionsnadelsystem nach einem der vorhergehenden Patentansprüche, mit einem Lithotripter zum Desintegrieren eines Nierensteins.

## Claims

1. Puncture-needle system for puncturing an organ or vessel, in particular a kidney, comprising a puncture cannula (6) which is inserted into a functional shaft (4) and is penetrated by an optical system (8), wherein the functional shaft (4) has an external diameter of less than 5 Fr.

2. Puncture-needle system according to claim 1, wherein the functional shaft (4) and the cannula (6) are connected by a coupling, preferably a Luer coupling (16, 22).

3. Puncture-needle system according to either of the preceding claims, comprising a grip (2) which is attached to the cannula (6) and/or the functional shaft (4) and comprises a Y-adapter (12) for attaching the optical system (8).

4. Puncture-needle system according to claim 3, comprising a T-adapter (24) which is intended for axially attaching to the Y-piece (12) and has a radial port (30) as a flush port.

5. Puncture-needle system according to claim 4, comprising an axial port (28) for a laser fibre or the like.

6. Puncture-needle system according to any one of the preceding claims, wherein an optical shifter (56) is associated with the optical system (8).

7. Puncture-needle system according to any one of the preceding claims, wherein the optical system (8) is designed to have an opening angle of more than 70°.

8. Puncture-needle system according to any one of the preceding claims, wherein the optical system (8) is designed to have a resolution of more than 6000 pixels.

9. Puncture-needle system according to any one of the preceding claims, wherein the functional shaft (4), the puncture cannula (6) and the grip (2), as well as the optical shifter (56), are designed to be disposable.

10. Puncture-needle system according to any one of claims 1 to 9, wherein the grip (2) can be autoclaved.

11. Puncture-needle system according to any one of the preceding claims, comprising an additional functional shaft (4) having a larger diameter and an obturator (70) for bougienage of the access opening produced by the puncture cannula (6) and having a guide wire for guiding the functional shaft (4).

12. Puncture-needle system according to any one of the preceding claims, comprising a lithotripter for disintegrating a kidney stone.

## Revendications

1. Système d'aiguille de ponction pour la ponction d'un organe ou d'un vaisseau, en particulier d'un rein, avec une canule de ponction (6) qui est insérée dans une tige de travail (4) et traversée par une optique (8), dans lequel la tige de travail (4) a un diamètre externe inférieur à 5 Fr.

2. Système d'aiguille de ponction selon la revendication 1, dans lequel la tige de travail (4) et la canule (6) sont reliées par un couplage, de préférence un couplage Luer (16, 22).

3. Système d'aiguille de ponction selon l'une des revendications précédentes, avec une poignée (2) qui est fixée à la canule (6) et/ou à la tige de travail (4) et qui a un adaptateur en Y (12) pour la fixation de l'optique (8).

4. Système d'aiguille de ponction selon la revendication 3, avec un adaptateur en T (24) pour la fixation axiale à la pièce en Y (12) avec un raccordement radial (30) comme raccordement de rinçage.

5. Système d'aiguille de ponction selon la revendication 4, avec un raccordement axial (28) pour une fibre laser ou similaire.

6. Système d'aiguille de ponction selon l'une des revendications précédentes, dans lequel un système de décalage d'optique (56) est affecté à l'optique (8).

7. Système d'aiguille de ponction selon l'une des revendications précédentes, dans lequel l'optique (8) est conçue avec un angle d'ouverture de plus de 70°.

8. Système d'aiguille de ponction selon l'une des revendications précédentes, dans lequel l'optique (8) est conçue avec une résolution de plus de 6000 pixels.

9. Système d'aiguille de ponction selon l'une des revendications précédentes, dans lequel la tige de travail (4), la canule de ponction (6) et la poignée (2) ainsi que le système de décalage d'optique (56) sont conçus de façon à être jetables.

10. Système d'aiguille de ponction selon l'une des revendications 1 à 9, dans lequel la poignée (2) est conçue de façon à être autoclavable.

11. Système d'aiguille de ponction selon l'une des revendications précédentes, avec une autre tige de travail (4) avec un diamètre supérieur et un obturateur (70) pour dilater l'accès produit avec la canule de ponction (6) et avec un fil-guide pour guider la tige de travail (4).

12. Système d'aiguille de ponction selon l'une des revendications précédentes, avec un lithotripteur pour désintégrer un calcul rénal.
